# EUROPÄISCHE PATENTSCHRIFT

(11) **EP 0 369 168 B1**
(45) Veröffentlichungstag und Bekanntmachung des Hinweises auf die Patenterteilung: **02.02.1994**
(21) Anmeldenummer: 89118990.4
(22) Anmeldetag: 12.10.1989
(51) Int. Cl.: G01N 33/49

(54) **Verfahren und Vorrichtung zum Untersuchen und Messen der Blutgerinnungszeit**
Method and apparatus for investigating and measuring the clotting time of blood
Procédé et appareil pour étudier et mesurer le temps de coagulation du sang

(30) Priorität: 31.10.1988 DE 3837078
(43) Veröffentlichungstag der Anmeldung: 23.05.1990
(73) Patentinhaber: Behnk, Holger, D-22417 Hamburg (DE)
(72) Erfinder: Behnk, Holger, D-22417 Hamburg (DE)
(74) Vertreter: Glawe, Delfs, Moll & Partner

(56) Entgegenhaltungen:
- WO-A-87/02131
- FR-A- 2 383 444
- US-A- 3 233 975
- US-A- 3 504 376

## Beschreibung

Die Erfindung betrifft ein Verfahren und eine Vorrichtung zum Untersuchen und Messen der Blutgerinnungszeit, bei dem Blut oder Blutplasma und ein Reagenz in eine Meßküvette eingebracht werden und die Gerinnungszeit elektrooptisch mit Hilfe eines magnetisch anziehbaren, in der Meßküvette angeordneten Rührgliedes, das durch eine außerhalb der Meßküvette angeordnete Magnetrühreinrichtung angetrieben wird, gemessen wird.

Es ist ein Verfahren bekannt (US-A 3 233 975), bei dem Blutplasma und Reagenz in eine flache Küvette eingefüllt werden, die dann von Hand gekippt wird, so daß die Flüssigkeiten zusammenfließen. Dieses Verfahren ist nur von Hand durchzuführen, was hohen personellen Aufwand erfordert. Außerdem ist die Küvette sehr flach, so daß Kapillarwirkung auf die Flüssigkeiten ausgeübt wird, die die Mischung der Flüssigkeiten dann behindert.

Verfahren und Vorrichtungen der eingangs genannten Art (DE-PS 31 27 560, EP-PS 0 090 192) erlauben sehr vielfältige und verschiedene Arten der Messung der Gerinnungszeit. Man erhält zwar sehr genaue Werte, es ist aber ein hoher Bedienungsaufwand durch geschulte Kräfte erforderlich.

Es sind zwar automatische Verfahren zur Messung der Blutgerinnungszeit bekannt, die aber nicht für das eingangs genannte Meßverfahren mit Rührgliedern anwendbar sind. Außerdem haben diese Verfahren schwerwiegende Nachteile. Diese Nachteile stehen mit der Tatsache in Zusammenhang, daß einerseits das Blutplasma und die Reagenzien vor der Messung gekühlt werden müssen, während andererseits die Messung bei einer Temperatur stattfinden muß, die nur sehr kleine Abweichungen von 37°C haben darf. Werden die Reagenzien nicht bei einer Temperatur von 15°C aufbewahrt, so zerfallen sie zum Teil schon nach einer halben Stunde. Weicht andererseits die Temperatur bei der Messung nur um 1° von 37°C ab, so erhält man bereits einen Meßfehler von 10%.

Bei einem automatischen Verfahren zum Untersuchen und Messen der Blutgerinnungszeit (Prospekt MLA Electra 800® der AHS/Deutschland GmbH) werden die mit Blutplasma vorher von Hand befüllten Meßküvetten in ein Karussell eingesetzt, in dem sie temperiert werden. Das Reagenz wird dann durch eine Pumpe aus dem gekühlten Vorratsbehälter durch ein plattenförmiges Element, in dem eine Erwärmung auf 37°C stattfindet, in die Küvette eingefüllt, wenn diese sich an der geeigneten Station befindet. Hier treffen dann das auf 37°C erwärmte Plasma und das ebenfalls erwärmte Reagenz zusammen. Der Nachteil besteht einmal darin, daß das Plasma immer noch von Hand in die Meßküvetten eingefüllt werden muß und daß diese Meßküvetten dann von Hand in das Karussell eingesetzt werden müssen. Der weitere Nachteil besteht darin, daß die plattenförmigen Wärmeaustauscher verhältnismäßig viel Reagenz aufnehmen. Bei Unterbrechung der Messung muß dann das Reagenz aus diesen plattenförmigen Wärmeaustauschern zurückgepumpt werden, da es sonst zerfällt. Anschließend trocknen aber Restbestände an den Wänden an, so daß bei Wiederinbetriebnahme die Gefahr von Verstopfungen oder Fehlmessungen besteht. Die Wärmetauscherplatten müssen daher häufig ausgewechselt werden. Weiter besteht die Gefahr von Verlusten der verhältnismäßig teueren Reagenzflüssigkeit. Außerdem ist für jedes Reagenz eine separate Pumpe und eine separate Wärmetauscherfläche notwendig, so daß bei dem genannten Gerät nur gleichzeitig zwei Reagenzien für eine Messung verwendet werden können. Diese Zahl der Reagenzien kann ohne übermäßigen Aufwand nicht beliebig erhöht werden.

Bei einem weiteren Verfahren (Prospekt COAC-A-Mate-X2® der Firma Labordiagnostica Gödecke) wird auf die plattenförmigen Wärmetauscher verzichtet. Stattdessen werden die Schläuche für die Reagenzien durch ein plattenförmiges Element geführt, das die Temperatur von 37°C hat. Hier können und/oder müssen die Schläuche dann nach Unterbrechung der Messung gereinigt oder ausgewechselt werden, was ebenfalls umständlich und teuer ist; es besteht auch leicht die Gefahr von Fehlmessungen.

Bei einem weiteren vorbekannten Verfahren (Prospekt ACL® Automated Coagulation Laboratory von Instrumentation Laboratory) wird das Plasma automatisch aus einem Zentrifugenröhrchen in eine Küvette eingeführt. Auch die Reagenzien werden automatisch aus gekühlten Vorratsbehältern in die Meßküvette eingefüllt. Die Übertragungseinrichtung kann dabei zwischendurch noch einer Wascheinrichtung zugeführt werden. Anschließend wird der Raum mit den auf einer Zentrifuge angeordneten Meßküvetten verschlossen. Die Meßküvetten werden dann erwärmt und nach Erreichen der Temperatur von 37°C in schnelle Rotation versetzt, wobei durch die Zentrifugenwirkung eine Durchmischung erfolgt. Dieses System hat ebenfalls verschiedene Nachteile.

In der Zentrifuge sind einmal nicht alle Arten von Messungen möglich. Es können keine Reagenzien verwendet werden, die sedimentieren, z.B. PTT Reagenz mit Kaolin. Auch die vorteilhaften Messungen mit Rührgliedern sind nicht möglich. Die Küvetten müssen auch unbedingt dicht sein, da sonst bei der hohen Zentrifugiergeschwindigkeit von 1200 Umdrehungen/Minute Flüssigkeit in den Rotorraum dringt und denselben verschmutzt, insbesondere auch die Meßeinrichtungen verschmutzt. Schließlich können die Meßküvetten nach der Messung nicht automatisch entsorgt werden, sondern müssen von Hand herausgenommen werden.

Ähnliche Probleme treten bei einem weiteren vorbekannten Verfahren (WO-A 87 02 131) auf. Die Messung muß durchgeführt werden, während die Probe rotiert und ist daher kompliziert. Eine gute Durchmischung kann nicht erreicht werden. Das Verfahren kann auch nicht mit Reagenzien angewendet werden, die sedimentierende Bestandteile aufweisen. Außerdem ist nicht klar, wie das Reagenz in den äußeren Teil der Küvette eingefüllt werden soll. Weist die Küvette hier ein Loch auf, so dürfte während des Zentrifugiervorgangs Reagenz und/oder Plasma herausspritzen.

Die Aufgabe der Erfindung besteht in der Schaffung eines Verfahrens der eingangs genannten Art, bei dem unter Ausnützung der Vorteile des Verfahrens mit einem Rührglied sehr viel verschiedene Untersuchungen mit unterschiedlichen Reagenzien mit schnellem Wechsel von einem Reagenz zum anderen möglich ist, wobei das Verfahren besonders für automatische Anwendungen ausgebildet ist.

Die erfindungsgemäße Lösung besteht darin, daß Blutplasma und Reagenz nebeneinander auf eine im wesentlichen horizontale Innenfläche einer über diese Fläche mit einer Öffnung versehenen Meßküvette eingebracht werden, daß die Meßküvette und ihr Inhalt auf die Reaktionstemperatur erwärmt werden, daß die Meßküvette in der Meßstation um im wesentlichen 90° so geschwenkt wird, daß die Innenfläche im wesentlichen senkrecht steht und Plasma und Reagenz zusammenfließen, und daß anschließend die Messung durchgeführt wird.

Blutplasma und Reagenz werden also nebeneinander auf eine im wesentlichen horizontale Fläche aufgebracht; sie haben dabei zunächst noch die Temperatur von z.B. 15°C, bei der noch keine Reaktionen auftreten. Anschließend werden dann die Meßküvette und ihr Inhalt auf die Reaktionstemperatur erwärmt, wobei aber noch keine Reaktionen zwischen Plasma und Reagenz stattfinden, da die beiden Flüssigkeiten nebeneinander angeordnet sind und sich noch nicht vermischt haben. Anschließend wird dann die Meßküvette um im wesentlichen 90° so geschwenkt, daß die Innenfläche im wesentlichen senkrecht steht, wodurch Plasma und Reagenz zusammenfließen. Anschließend kann dann die Messung durchgeführt werden.

Die Befüllung der Meßküvetten mit Plasma und Reagenzien kann dabei automatisch mit nur einer Pumpe erfolgen. Irgendwelche Verschmutzungsprobleme, Inkubationsprobleme, Verfallsprobleme und dergleichen ergeben sich nicht, da die Pumpe und die Zuführeinrichtungen gekühlt sein können. Die Meßküvette kann dann automatisch in eine Erwärmungsstation geführt werden, wo sie so lange erwärmt wird, bis sie die Temperatur von 37°C erreicht hat. Anschließend kann dann die Messung durchgeführt werden, wobei die Vorteile des Verfahrens mit einem Rührglied ausgenützt werden können. Anschließend erfolgt dann die automatische Entsorgung. Selbstverständlich ist es aber auch möglich, das Verfahren von Hand durchzuführen, wenn eine entsprechende Vorrichtung zur automatischen Durchführung des Verfahrens nicht vorhanden ist.

Wird die Innenfläche anfangs um einige Grad so geneigt, daß der bei der Schwenkung nach oben zu schwenkende Endbereich der Innenfläche tiefer liegt als die übrigen Bereiche der Innenfläche, so kann anfangs das Rührglied in diesem tieferen Bereich angeordnet werden. Man kann dabei zunächst die Küvette neigen und dann das Rührglied in den tieferliegenden Bereich einbringen oder aber das Rührglied erst einbringen, so daß es beim anschließenden Neigen an die gewünschte Stelle rollt. Das Rührglied fällt dann bei Beginn des Schwenkvorgangs in das Reagenz und dann in das Plasma hinein und zieht diese mit nach unten, wodurch eine bessere Durchmischung bereits am Anfang erreicht wird. Gleichzeitig sorgt das Rührglied für eine konstante Geschwindigkeit beim Transport des Reagenzes.

Bei anderen Ausführungsformen wird man die Neigung im genau umgekehrten Sinne wählen, damit das Rührglied beim Schwenken nicht durch die Flüssigkeiten hindurchfällt, was zu Spritzern und unerwünschter Verteilung der Flüssigkeiten führen könnte.

Als besonders zweckmäßig hat es sich erwiesen, wenn das Rührglied eine Metallkugel ist.

Läßt man nach der Schwenkung die Meßküvette eine begrenzte Strecke fallen und auf eine Anschlagfläche auftreffen, so werden Rührglied und Flüssigkeiten stoßartig nach unten bewegt, so daß schnell maximale Mengen der Flüssigkeiten hier vorhanden sind und durchmischt werden können.

Es ist ohne weiteres möglich, das Verfahren fließbandartig so auszugestalten, daß gleichzeitig mehrere Meßküvetten durch die einzelnen Stationen in aufeinanderfolgende Reihenfolge geführt und anschließend entsorgt werden.

Dies ist insbesondere möglich bei einer Meßküvette zum Untersuchen und Messen der Blutgerinnungszeit, die mit einer im wesentlichen horizontal angeordneten Innenfläche, einer Öffnung über dieser Innenfläche und einer ein Zusammenfließen der Flüssigkeiten behindernden Oberflächenstruktur versehen ist, die sich dadurch auszeichnet, daß sie ein magnetisch anziehbares Rührglied aufweist und daß mehrere Meßküvetten nebeneinander in einer Halterung angeordnet sind, die eine Zahnstange aufweist.

Da mehrere Meßküvetten in einer gemeinsamen Halterung angeordnet sind, die eine Zahnstange aufweist, so können diese mehreren Meßküvetten durch einen Zahnradantrieb durch unterschiedliche Stationen geführt werden. Auf diese Weise kann auf sehr rationelle Weise eine große Zahl von Untersuchungen in schneller Folge durchgeführt werden.

Dadurch, daß die Innenfläche im wesentlichen eben horizontal anzuordnen ist, können besonders große Mengen von Plasma und Reagenz nebeneinander angeordnet werden, die aber durch die Oberflächenstrukturen daran gehindert werden, bereits in dieser Stellung der Meßküvette zusammenzufließen. Werden die Meßküvetten anschließend geschwenkt, insbesondere um ungefähr 95°, so daß sie anschließend senkrecht stehen, so können diese Oberflächenstrukturen ein Zusamßenfließen nicht mehr behindern. Dies gilt insbesondere dann, wenn man die Küvette nach dem Schwenken noch auf eine Anschlagfläche herabfallen läßt, wobei hier eine Wegstrecke von 5 mm bereits ausreicht.

Die Oberflächenstrukturen können kleine wannenartige Vertiefungen für die Flüssigkeiten sein.

Zweckmäßigerweise werden durch die Oberflächenstrukturen durch eine Zwischenfläche voneinander getrennte Bereiche begreuzt. Die Flüssigkeiten verbleiben dabei zunächst in den Oberflächenbereichen und sind durch die Zwischenfläche voneinander getrennt. Die Oberflächenstrukturen können linienförmige, gratartige Vorsprünge sein. Besonders einfach sind die Oberflächenstrukturen aber herzustellen, wenn sie linienförmige Einkerbungen sind.

Wenn die Innenfläche im zunächst tieferliegenden Bereich an ihrem Rand durch zwei in der Mitte stumpfwinklig zusammenlaufende Begrenzungswände begrenzt ist, so wird ein kugelförmiges Rührglied am Anfang automatisch in die Mitte der Kante rollen, so daß es dann von dieser Mitte durch die Flüssigkeitstropfen hindurchfällt und dadurch besonders viel Flüssigkeiten mit in den Bereich mitnimmt, in dem die anschließenden Messung durchgeführt werden soll. An der Fläche, auf die das Rührglied und die Flüssigkeit treffen, ist zweckmäßigerweise eine mittige zylinderförmige Vertiefung vorgesehen, so daß hier das kugelförmige Rührglied eine kreisförmige Bewegung durchführen kann, die durch die Magnetrühreinrichtung bewirkt wird.

Zweckmäßigerweise wird vorgesehen, daß die außerhalb der zylinderförmigen Ausnehmung angeordneten Randbereiche nach innen und in der Stellung nach der Schwenkung nach unten abgeschrägt sind, wobei wenigstens teilweise diese Endbereiche eine geringere Dicke als der Durchmesser der Kugel haben. Durch diese abgeschrägten, insbesondere kugelschalenförmig abgeschrägten Bereiche wird sichergestellt, daß die Kugel auch dann schnell in die vorgesehene zylinderförmige Bahn gelangt, falls sie auf die Randbereiche auftrifft. Wenn die Endbereiche eine geringere Dicke als der Durchmesser der Kugel haben, so wird durch die Kugel nicht nur das Flüssigkeitsmaterial im zylinderförmigen Bereich durchrührt; vielmehr wird auch auf die Flüssigkeitsteile eine Rührwirkung ausgeübt, die sich in den Randbereichen, insbesondere in den Ecken einer rechteckigen Meßküvette befinden.

Verfahren und Vorrichtung haben wie bereits erwähnt den Vorteil, daß gleichzeitig sehr viele verschiedene Messungen, nämlich insgesamt bis zu 13 Bestimmungen möglich sind. Es ist leicht möglich, die entsprechenden 13 Reagenzien gekühlt zu lagern und jederzeit einen kompletten Gerinnungsstatus zu machen. Während man bei den vorbekannten Automaten drei Pumpen z.B. für die Grundbestimmungen PT (prothrombin time = Prothrombinzeit) und PTT (partial thrombin time = partielle Thrombinzeit) hatte, die lange Zeit für den Gerinnungsstatus gereicht haben, ist es jetzt auch möglich, zusätzlich TT (thrombin time = Thrombinzeit) und Fibrinogen zu messen, was bereits in vielen Krankenhäusern geschieht. Mit keinem vorbekannten Gerät kann dies pro Patient in einem Durchgang gemessen werden. Sollten sich bei diesem Gerinnungstatus Fehler ergeben, so können und müssen zusätzlich noch die Faktoren gemessen werden. Dies summiert sich dann zu den erwähnten insgesammt 13 Bestimmungen.

Die Erfindung wird im folgenden anhand einer vorteilhaften Ausführungsform unter Bezugnahme auf die beigefügten Zeichnungen beschrieben. Es zeigen:
- Fig.1:: Das Blockschema eines vorbekannten automatischen Verfahren;
- Fig.2:: Das Blockschema des erfindungsgemäßen Verfahrens;
- Fig.3:: Das Prinzip der Verwendung der erfindungsgemäßen Meßküvette;
- Fig.4:: Die Meßküvette im liegenden Zustand in Draufsicht;
- Fig.5:: Diesselbe Meßküvette in Endansicht;
- Fig.6:: Die Meßküvette in der Meßstation, wobei die Meßküvette gegenüber der Darstellung von Fig.4 um 90° um ihrer Längsachse gedreht gezeigt ist; und
- Fig.7:: eine Anzahl von Meßküvetten, die in einer gemeinsamen Halterung mit einer Zahnstange zusammengefaßt sind.

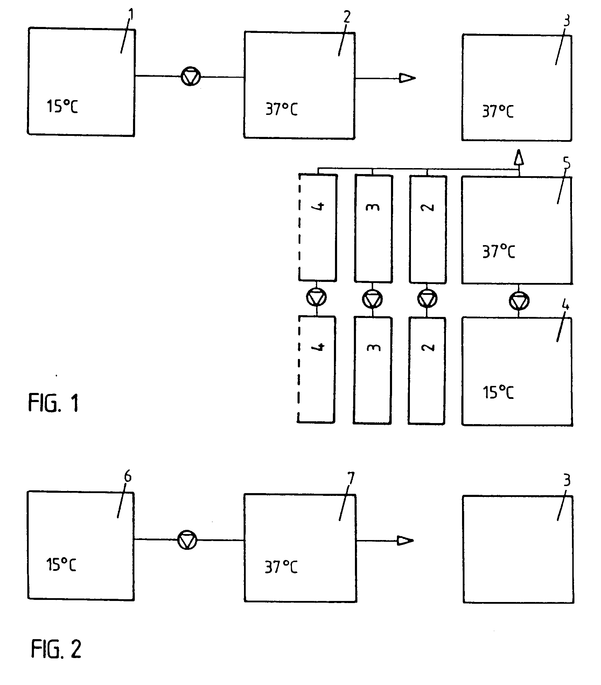

Im Blockschema des vorbekannten Verfahrens (Prospekt MLA Elektra 800® und COAG-A-MATE®) der Fig.1 wird zunächst im Schritt 1 das Plasma und Reagenz bei einer Temperatur von 15°C aufbewahrt. Im Schritt 2 erfolgt dann über Oberflächenkontakt mit dem Inkubator die Inkubation auf 37°C. Anschließend wird dann das so erwärmte Plasma bzw. Plasma/Reagenzgemisch der Meßstation 3 zugeführt. Andererseits wird das Reagenz zunächst in einer Station bei einer Temperatur von 15°C aufbewahrt. Es erfolgt dann in der Station 5 in plattenförmigen Wärmetauschern oder den Schläuchen die Inkubation auf 37°C. Anschließend wird dann auch das Reagenz in die Meßposition 3 verbracht.

Es wird also bei der Automatisierung eine Pumpe für Plasma und Reagenz in Schritt 1 benötigt. In Schritt 3 wird für jedes Reagenz eine Pumpe sowie ein Wärmetauscher benötigt.

Das in Fig.2 dargestellte erfindungsgemäße Verfahren ist demgegenüber wesentlich einfacher. Hier werden Plasma und Reagenz nebeneinander in die Meßküvette bei einer Temperatur von 15°C eingebracht. Anschließend erfolgt dann in Schritt 7 die Inkubation auf eine Temperatur von 37°C. Nach Schwenken der Meßküvette erfolgt dann die Messung in der Meßposition 3.

Es wird bei Automatisierung nur noch eine Pumpe für Plasma und Reagenz in Schritt 1 benötigt.

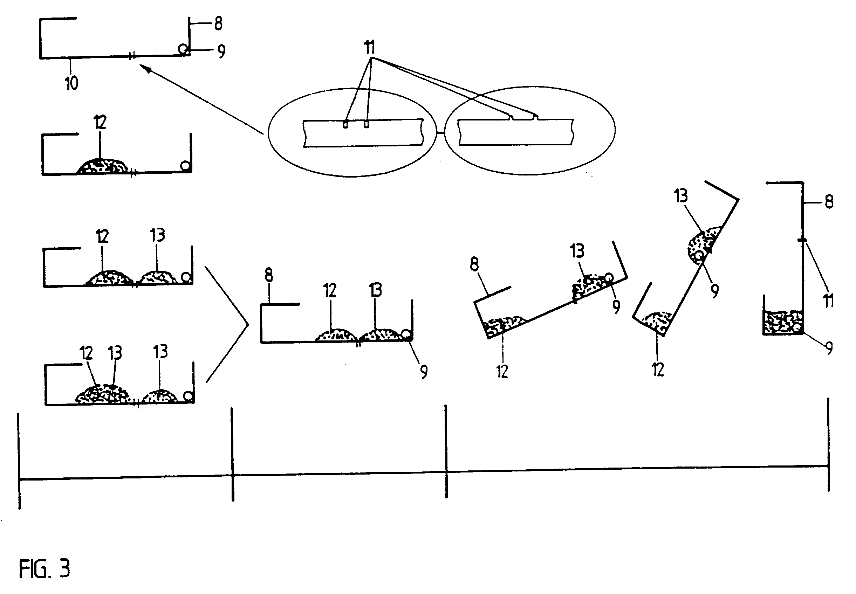

Der erfindungsgemäße Vorgang ist in Fig.3 deutlicher gezeigt, wobei die Meßküvette 8 in einer Seitenansicht im Schnitt gezeigt ist.

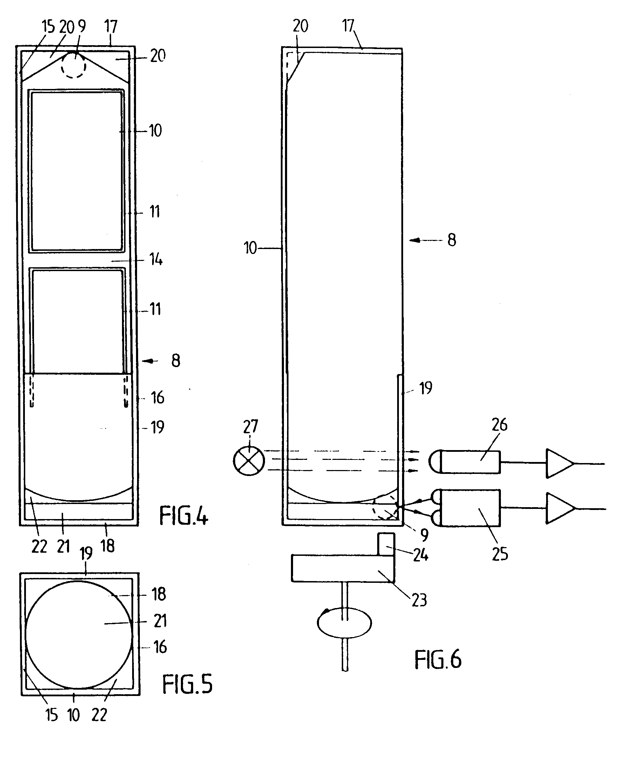

Die Meßküvette 8 ist im wesentlichen quaderförmig und besitzt an einer ihrer Flächen eine Öffnung, die den wesentlichen Teil dieser Flächen einnimmt. Die Meßküvette 8 hat also eine schuhähnliche Form. In Schritt 6 wird zunächst das Rührglied 9 in Form einer Kugel eingebracht. Die Meßküvette 8 ist dabei ein wenig geneigt, und zwar so, daß die Kugel 9 sich an der untersten Stellung befindet. Diese Neigung der Küvette 8 ist nicht unbedingt erforderlich und in Fig.3 auch nicht dargestellt. In der Mitte ist die untere Fläche 10 der Küvette 8 durch eingeritzte Vertiefungen 11 oder gratartige Vorsprünge unterteilt, die im Zusammenhang mit Fig.4 noch genauer beschrieben werden. Diese Vertiefungen bzw. Vorsprünge 11 sind in Fig. 3 auch noch vergrößert dargestellt. Links von den Einritzungen 11 wird das Plasma 12 eingebracht. Anschließend wird (in der Darstellung der Fig.3 von oben nach unten) rechts vom Plasma 12 und den Einritzungen 11 ein Reagenz 13 eingebracht. Falls erforderlich kann dem Plasma 12 anschließend noch ein weiteres Reagenz zugeführt werden.

Die Meßküvette 8 wird in diesem Zustand in eine weitere Station verbracht und im Schritt 7 auf eine Temperatur von 37°C inkubiert. Bei Erreichen der gewünschten Temperatur erfolgt dann im Schritt 3, d.h. in der Meßstation, das Kippen der Küvette 8. Wie dies im mittleren Teil bei Schritt 3 zu sehen ist, dringt dabei die Kugel 9 in das Reagenz 13 ein und nimmt dieses mit, so daß sich in der rechten Stellung die Kugel 9 unten befindet und dabei Plasma 12 und Reagenz 13 vermischt sind. Hier erfolgt dann die Messung der Gerinnungszeit.

Die Küvette 8 ist in Fig.4 in Draufsicht deutlicher gezeigt. Die in der Stellung von Schritt 6 und 7 von Fig.3 untere Fläche 10, auf die Plasma 12 und Reagenz 13 aufgebracht werden, ist mit rechtwinklig zueinanderstehenden Einkerbungen versehen, die zumindestens in der Fig.4 im oberen Bereich eine geschlossene Fläche umranden. Die beiden Bereiche, die durch die Einkerbungen 11 wenigstens teilweise umrandet sind, sind durch einen Zwischenbereich 14 getrennt, so daß die Flüssigkeiten, deren Fließen durch die Einkerbungen 11 behindert wird, deutlich voneinander getrennt sind, solange die Meßküvette 8 ihre im wesentlichen waagerechte Stellung einnimmt.

Die Seitenwände 15 und 16 sind geschlossen; ebenso die Stirnflächen 17 und 18. Ein Teil der oberen Fläche ist durch eine Abdeckung 19 verschlossen.

In der Fig.4 oben ist die Grundfläche 10 durch Schrägflächen 20 begrenzt, so daß die Kugel 9 in der Mitte der Fläche 10 angeordnet ist, wenn die Küvette 8 in diesem Bereich etwas tiefer geneigt ist. Die gegenüberliegende Stirnfläche 18 weist eine zylindrische Ausnehmung 21 auf, wobei die Randbereiche 22 in den Ecken abgeschrägt sind, wie dies auch aus Fig.4 ersichtlich ist. Fig.5 stellt dabei die Stirnfläche 18 in Draufsicht dar.

Durch die Schrägflächen 22 wird bewirkt, daß die Kugel in die zylindrische Ausnehmung 21 fällt, wenn die Meßküvette 8 in die senkrechte Stellung der Fig.6 geschwenkt wird. Die Kugel 9 ragt dabei bis in den Raum oberhalb der zylindrischen Ausnehmung 21 hinein, so daß sie die gesamte im unteren Bereich nach dem Schwenken befindliche Flüssigkeit gut durchmischt, wenn sie durch ein Magnetrührwerk 23 mit einem Permanentmagneten 24 bewegt wird.

Der Beginn der Gerinnung kann durch photoelektrische Einrichtungen bestimmt werden, die bei 25 und 26 schematisch dargestellt sind. Die Einrichtung 25 ist dabei eine Reflektions-Messung, während die Einrichtung 26 mit einer Lichtquelle 27 eine Transmissionsmeßeinrichtung ist. Diese Meßeinrichtungen sind aus den eingangs genannten Patentschriften bekannt, so daß es nicht notwendig ist, sie hier näher zu beschreiben. Es versteht sich, daß die Meßküvette durchsichtig ist, damit die Messungen durchgeführt werden können.

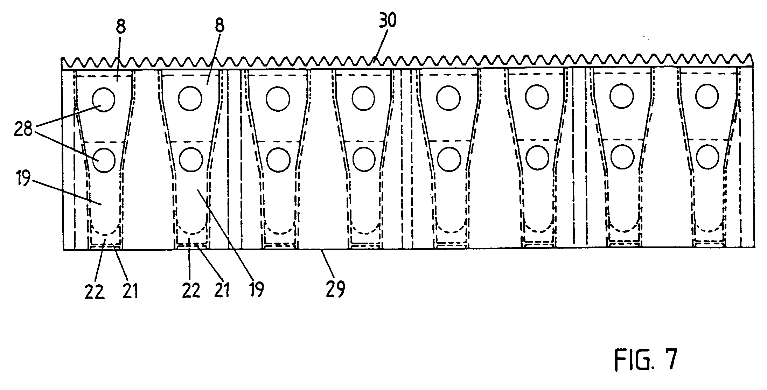

In Fig. 7 ist gezeigt, daß eine Reihe von Meßküvetten 8 nebeneinander in einer Halterung 29 angeordnet sind, die an ihrer Außenseite eine Zahnstange 30 trägt. Mit Hilfe dieser Zahnstange 30 und nicht gezeigter Zahnradantriebe kann die Halterung mit den Meßküvetten durch die einzelnen Stationen transportiert werden, so daß in schneller Folge eine große Anzahl von Untersuchungen durchgeführt werden kann. Bei der in Fig.7 gezeigten Ausführungsform überdeckt die Abdeckplatte 19 auch mehr oder weniger den gesamten Bereich der Meßküvette 8; die Abdeckplatte 19 weist lediglich zwei Öffnungen 28 auf, durch die Plasma, Reagenz und Kugel eingegeben werden können.

## Patentansprüche

1. Verfahren zum Untersuchen und Messen der Blutgerinnungszeit, bei dem Blut oder Blutplasma und ein Reagenz in eine Meßküvette eingebracht werden und die Gerinnungszeit elektrooptisch mit Hilfe eines magnetisch anziehbaren, in der Meßküvette angeordneten Rührgliedes, das durch eine außerhalb der Meßküvette angeordnete Magnetrühreinrichtung angetriebenwird, gemessen wird, dadurch gekennzeichnet, daß Blutplasma und Reagenz nebeneinander auf eine im wesentlichen horizontale Innenfläche einer über dieser Fläche mit einer Öffnung versehenen Meßküvette eingebracht werden, daß die Meßküvette und ihr Inhalt auf die Reaktionstemperatur erwärmt werden, daß die Meßküvette in der Meßposition um im wesentlich 90° so geschwenkt wird, daß die Innenfläche im wesentlichen senkrecht steht und Plasma und Reagenz zusammenfließen, und daß anschließend die Messung durchgeführt wird.

2. Verfahren nach Anspruch 1, dadurch gekennzeichnet, daß die Innenfläche anfangs um einige Grad geneigt wird.

3. Verfahren nach Anspruch 1 oder 2, dadurch gekennzeichnet, daß als Rührglied eine Metallkugel verwendet wird.

4. Verfahren nach einem der Ansprüche 1 bis 3, dadurch gekennzeichnet, daß nach der Schwenkung die Meßküvette eine begrenzte Strecke fallengelassen wird und auf eine Anschlagfläche auftrifft.

5. Verfahren nach einem der Ansprüche 1 bis 4, dadurch gekennzeichnet, daß die Vorgänge automatisch ablaufen und die Meßküvette durch eine Füllstation zum Einbringen von Plasma und Reagenz, eine Aufwärmstation und eine Meßstation zu einer Entsorgungsstation geführt wird.

6. Verfahren nach Anspruch 5, dadurch gekennzeichnet, daß gleichzeitig mehrere Meßküvetten durch die Stationen abgeführt werden.

7. Meßküvette zum Untersuchen und Messen der Blutgerinnungszeit, die mit einer im wesentlichen horizontal anzuordnenden Innenfläche, einer Öffnung über dieser Innenfläche und einer ein Zusammenfließen der Flüssigkeiten (12, 13) behindernden Oberflächenstruktur (11) versehen ist, dadurch gekennzeichnet, daß sie ein magnetisch anziehbares Rührglied aufweist und daß mehrere Meßküvetten (8) nebeneinander in einer Halterung (29) angeordnet sind, die eine Zahnstange (30) aufweist.

8. Meßküvette nach Anspruch 7, dadurch gekennzeichnet, daß die Oberflächenstrukturen Vertiefungen oder kleine wannenförmige Bereiche sind.

9. Meßküvette nach Anspruch 7, dadurch gekennzeichnet, daß die Oberflächenstrukturen (11) linienförmig sind und durch diese Strukturen durch eine Zwischenfläche (14) voneinander getrennte Bereiche begrenzt sind.

10. Meßküvette nach Anspruch 7 oder 9, dadurch gekennzeichnet, daß die Oberflächenstrukturen (11) linienförmige Vorsprünge sind.

11. Meßküvette nach Anspruch 7 oder 9, dadurch gekennzeichnet, daß die Oberflächenstrukturen (11) linienförmige Einkerbungen sind.

12. Meßküvette nach einem der Ansprüche 7 bis 11, dadurch gekennzeichnet, daß die Innenfläche (10) im zunächst tiefer liegenden Bereich an ihrem Rand durch zwei in der Mitte stumpfwinklig zusammenlaufende Kanten (bei 20) begrenzt ist.

13. Meßküvette nach Anspruch 12, dadurch gekennzeichnet, daß sie auf der entgegengesetzten Seite in der zur Innenfläche senkrechten, nach der Schwenkung horizontalen Endfläche (18) eine mittige zylinderförmige oder ovale Vertiefung (21) aufweist.

14. Meßküvette nach Anspruch 13, dadurch gekennzeichnet, daß die außerhalb der zylinderförmigen Ausnehmung (21) angeordneten Randbereiche (22) nach innen und in der Stellung nach der Schwenkung nach unten abgeschrägt sind, wobei wenigstens teilweise diese Endbereiche (22) eine geringere Dicke als der Durchmesser der Kugel (9) (Rührglied) haben.

## Claims

1. A method of investigating and measuring blood coagulation time, wherein blood or blood plasma and a reagent are introduced into a measuring cuvette and the coagulation time is measured electro-optically by means of an agitating member which can be attracted magnetically and which is disposed in the measuring cuvette and which is driven by a magnetic agitating device disposed outside the measuring cuvette, characterised in that blood plasma and reagent are introduced together on to a substantially horizontal inner surface of a measuring cuvette provided with an opening above this surface, in that the measuring cuvette and its contents are heated to the reaction temperature, in that in the measuring position the measuring cuvette is tilted through approximately 90° so that the inner surface is substantially vertical and the plasma and reagent flow together, and in that subsequently the measurement is carried out.

2. A method according to Claim 1, characterised in that the inner surface is initially inclined by a few degrees.

3. A method according to Claim 1 or 2, characterised in that a metal ball is used as the agitating member.

4. A method according to any one of Claims 1 to 3, characterised in that after tilting the measuring cuvette is allowed to drop for a limited distance and encounters an abutment surface.

5. A method according to any one of Claims 1 to 4, characterised in that the operations proceed automatically and the measuring cuvette passes through a filling station for introducing plasma and reagent, a heating station and a measuring station, and then to waste disposal station.

6. A method according Claim 5, characterised in that a plurality of measuring cuvettes pass simultaneously through the stations.

7. A measuring cuvette for investigating and measuring blood coagulation time, which is provided with an inner surface to be arranged substantially horizontal, with an opening above this inner surface and with a surface structure (11) which impedes any flowing together of the fluids (12, 13), characterised in that it has an agitating member which can be attracted magnetically, and in that a plurality of measuring cuvettes (8) are disposed side by side in a mounting (29) which has a rack (30).

8. A measuring cuvette according to Claim 7, characterised in that the surface structures are recesses or small trough-like zones.

9. A measuring cuvette according to Claim 7, characterised in that the surface structures (11) are linear and zones separated from one another by an intermediate surface (14) are bounded by these structures.

10. A measuring cuvette according to Claim 7 or 9, characterised in that the surface structures (11) are linear projections.

11. A measuring cuvette according to Claim 7 or 9, characterised in that the surface structures (11) are linear indentations.

12. A measuring cuvette according to any one of Claims 7 to 11, characterised in that the inner surface (10) in the initially lower lying zone on its periphery is defined by two edges (at 20) converging centrally at an obtuse angle.

13. A measuring cuvette according to Claim 12, characterised in that on the opposite side in the end surface (18) perpendicular to the inner surface, which is horizontal after tilting, there is a central cylindrical or oval recess (21).

14. A measuring cuvette according to Claim 13, characterised in that the peripheral zones (22) disposed outside the cylindrical recess (21) are inclined inwardly and in the position after tilting has taken place they are downwardly inclined, these end zones (22) being at least partly of smaller thickness than the diameter of the ball (9) ('agitating member').

## Revendications

1. Procédé pour l'étude et la mesure du temps de coagulation du sang, dans lequel du sang ou du plasma sanguin et un réactif sont placés dans une cuvette de mesure et le temps de coagulation est mesuré électrooptiquement à l'aide d'un organe agitateur susceptible d'attraction magnétique, disposé dans la cuvette de mesure et entraîné par un dispositif d'agitation magnétique disposé à l'extérieur de la cuvette de mesure, caractérisé en ce que le plasma sanguin et le réactif sont introduits côte à côte sur une surface intérieure, pratiquement horizontale, d'une cuvette de mesure munie d'une ouverture au-dessus de cette surface, en ce que la cuvette de mesure et son contenu sont récbauffés à la température de réaction, en ce que la cuvette de mesure est basculée pratiquement de 90° dans la position de mesure, de telle sorte que la surface intérieure soit pratiquement verticale et que le plasma et le réactif s'écoulent ensemble et en ce que la mesure est ensuite effectuée.

2. Procédé selon la revendication 1, caractérisé en ce qu'au début, la surface intérieure est inclinée de quelques degrés.

3. Procédé selon la revendication 1 ou 2, caractérisé en ce qu'une bille métallique est utilisée comme organe agitateur.

4. Procédé selon l'une quelconque des revendications 1 à 3, caractérisé en ce qu'après son basculement, la cuvette de mesure parcourt un trajet limité en chute libre et vient frapper une surface de butée.

5. Procédé selon l'une quelconque des revendications 1 à 4, caractérisé en ce que les processus se déroulent automatiquement et en ce que la cuvette de mesure est transportée entre un poste de remplissage pour l'introduction de plasma et de réactif, un poste de réchauffement et un poste de mesure, vers un poste de déchargement.

6. Procédé selon la revendication 5, caractérisé en ce que plusieurs cuvettes de mesure sont transportées en même temps entre les postes.

7. Cuvette de mesure pour l'étude et la mesure du temps de coagulation du sang, qui comporte une surface intérieure pratiquement horizontale, une ouverture au-dessus de cette surface intérieure et une structure de surface (11) empêchant les liquides (12,13) de s'écouler ensemble, caractérisée en ce qu'elle présente un organe agitateur susceptible d'attraction magnétique, et en ce que plusieurs cuvettes de mesure (8) sont disposées côte à côte dans un support (29) qui est muni d'une crémaillère (30).

8. Cuvette de mesure selon la revendication 7, caractérisée en ce que les structures de surface sont des creux ou de petites régions en forme d'auge.

9. Cuvette de mesure selon la revendication 7, caractérisée en ce que les structures de surface (11) sont linéaires et que des régions séparées l'une de l'autre par une surface intermédiaire (14) sont limitées par ces structures.

10. Cuvette de mesure selon la revendication 7 ou 9, caractérisée en ce que les structures de surface (11) sont des saillies linéaires.

11. Cuvette de mesure selon la revendication 7 ou 9, caractérisée en ce que les structures de surface (11) sont des entailles linéaires.

12. Cuvette de mesure selon l'une quelconque des revendications 7 à 11, caractérisée en ce que dans sa région située le plus bas initialement, la surface intérieure (10) est limitée au niveau de son bord, par deux arêtes (en 20) qui convergent à angle obtus au milieu.

13. Cuvette de mesure selon la revendication 12, caractérisée en ce qu'elle présente du côté opposé, dans la surface d'extrémité (18) qui est perpendiculaire à la surface intérieure et est disposée horizontalement après le basculement, un creux médian cylindrique ou ovale (21).

14. Cuvette de mesure selon la revendication 13, caractérisée en ce que les régions du bord (22) situées en dehors du creux cylindrique (21) sont biseautées vers l'intérieur et, dans la position après le basculement, vers le bas, ces régions d'extrémité (22) ayant, au moins en partie, une épaisseur plus petite que le diamètre de la bille (9) (organe agitateur).
